# EUROPÄISCHE PATENTSCHRIFT

(11) **EP 0 501 179 B1**
(45) Veröffentlichungstag und Bekanntmachung des Hinweises auf die Patenterteilung: **09.08.1995**
(21) Anmeldenummer: 92101774.5
(22) Anmeldetag: 04.02.1992
(51) Int. Cl.: A61M 5/44

(54) **Vorrichtung zum Erwärmen von medizinischen Flüssigkeiten**
Medical fluids heating device
Dispositif de chauffage des fluides médicaux

(30) Priorität: 23.02.1991 DE 4105781
(43) Veröffentlichungstag der Anmeldung: 02.09.1992
(73) Patentinhaber: Fresenius AG, 61350 Bad Homburg (DE)
(72) Erfinder: Evering-Hattisch, Hans-Gerd, W-6394 Grävenwiesbach (DE); Hansen, Walter, W-6380 Bad Homburg (DE); Schneider, Hans-Peter, W-6392 Neu Anspach 1 (DE); Polaschegg, Hans-Dietrich, Dr., W-6370 Oberursel 4 (DE); Steinbach, Bernd, Dr., W-6380 Bad Homburg (DE); Peter, Monika, W-6384 Schmitten 2 (DE)
(74) Vertreter: Fuchs Mehler Weiss

(56) Entgegenhaltungen:
- EP-A- 0 242 724
- EP-A- 0 247 989
- WO-A-90/07947
- DE-A- 3 545 338

## Beschreibung

Die Erfindung betrifft eine Vorrichtung zum Erwärmen von medizinischen Flüssigkeiten mit einem U-förmigen Metallrohr, einer elektrischen Energiequelle und mit einer Heizsteuerungseinheit gemäß der WO-A-90/07947.

Bei der Applizierung medizinischer Flüssigkeiten, beispielsweise von isotonischen Kochsalzlösungen, Plasmaexpanderlösungen, Substitutionslösungen u. dgl. ist darauf zu achten, daß diese auf die Körpertemperatur des Patienten erwärmt werden. Dies gilt insbesondere bei der Applikation erheblicher Flüssigkeitsmengen, die üblicherweise bei niedrigen Temperaturen aufbewahrt werden.

Bekannt sind dabei u. a. spiralförmige Schlauchsegmente, die in einem Warmwasserbad liegen, in dem der Wärmeaustausch realisiert wird.

Weiterhin kann die zu erwärmende Flüssigkeit durch einen Beutel mit mäanderförmiger Flüssigkeitsführung geleitet werden, wobei der Beutel zwischen zwei elektrisch beheizten Platten angeordnet ist, die den Beutel auf eine vorbestimmte Temperatur erwärmen (US-Patent 40 98 123).

Ein weiteres Prinzip besteht in der Verwendung einer Plattenheizvorrichtung, bei der anstelle des zu erwärmenden Beutels ein Schlauch um einen zylindrischen Wickelkörper gelegt ist. Zur Verbesserung des Wärmeübergangs ist dabei der Wickelkörper mit einer umlaufenden spiralförmigen Vertiefung versehen, in der der Schlauch mit passendem Durchmesser eingelegt werden kann.

Schließlich vertreibt die Fa. Engström Medical AB eine Heizvorrichtung, die ein direkt beheizbares Edelstahlrohr aufweist, durch das die zu erwärmende medizinische Flüssigkeit, insbesondere eine Substituatlösung, geführt wird.

Sämtliche bekannten Vorrichtungen weisen den Nachteil auf, daß sie nur bei einem bestimmten Fluidfluß die vorbestimmte Temperatur erreichen, jedoch bei Abweichungen hiervon nicht mehr den erforderlichen Temperaturbereich einhalten können bzw. nur langsam korrigierend arbeiten.

Aus der bereits eingangs erwähnten Druckschrift WO-A-90/07947 ist eine Vorrichtung mit den Merkmalen gemäß dem Oberbegriff des Anspruchs 1 bekannt geworden. In dieser Druckschrift ist ein U-förmiges Rohr beschrieben, welches über ein separates Heizelement erhitzt wird. Das U-förmige Rohr besteht in einem Ausführungsbeispiel aus einer Aluminiumlegierung, bei dem die Innenwandung mit einer besonderen Beschichtung (Aluminium-Telfa) versehen ist, um eine Wärmeleitung vom Heizelement über das U-förmige Rohr hin zu der das Rohr durchströmenden Flüssigkeit zu erhöhen.

Bei dieser bekannten Vorrichtung ist die Regelung der Temperatur der medizinischen Flüssigkeit recht träge. Dies erklärt sich allein schon daraus, daß erst das Heizelement selbst auf eine bestimmte Temperatur gebracht werden muß, bevor diese über das U-förmige Rohr der Flüssigkeit verliehen werden kann. Insbesondere bei sich ändernden Flußraten oder bei hohen Flüssen ist eine exakte Regelung mit dieser Vorrichtung nicht zu erwarten.

Vor diesem Hintergrund ist es die Aufgabe der vorliegenden Erfindung, eine gattungsgemäße Vorrichtung so weiterzubilden, daß eine vorbestimmte Flüssigkeitstemperatur bei konstanten und wechselnden Flußraten zwischen nahezu 0 und 250 ml/min. erreicht wird.

Die lösung der Aufgabe gelingt durch die kennzeichnenden Merkmale des Anspruchs 1.

Mit der erfindungsgemäßen Vorrichtung kann selbst bei wechselnden hohen Flußraten wie auch bei sehr geringen Flußraten eine vorbestimmte Flüssigkeitstemperatur, beispielsweise etwa 37° C, mit sehr geringen Abweichungen (± 1 %) eingehalten werden. Dabei wird gewahrleistet, daß sich die Flüssigkeit während des Transports durch die Erwärmung nicht in ihrer Substanz verändert.

Mit der erfindungsgemäßen Vorrichtung wird die jeweilige Flüssigkeit während der Applikation beim Durchströmen des Metallrohrs erwärmt. Dieses Rohr wird elektrisch beheizt, wobei es an eine Wechselstromquelle angeschlossen ist.

Beim Durchströmen durch ein aktiv heizendes Rohrsegment treten bei der Erwärmung der Flüssigkeit zwei verschiedene Wärmetransportmechanismen auf, nämlich die Wärmeleitung und die Wärmekonvektion. Aufgrund der Temperaturdifferenz in unmittelbarer Nähe der festen Rohrwand kommt es zum Wärmeübergang, während in einiger Entfernung hiervon der Wärmetransport mittels Konvektion strömungsmäßig erfolgt.

Die notwendige Wärmeenergie zur Erwärmung einer Flüssigkeit ergibt sich aus folgenden Eingangsgrößen: Volumenstrom, IST- und SOLL-Temperatur und Wärmekapazität der Flüssigkeit. Aufgrund dieser Eingangsgrößen ergeben sich in Abhängigkeit vom elektrischen Widerstand des Heizungsrohrs die Strom- und Spannungswerte. Diese Größen werden vorteilhafterweise aus sicherheitstechnischen Gründen möglichst klein gehalten. Insofern wird ein Rohr mit möglichst geringer Wandstärke, beispielsweise etwa 0,1 mm gewählt. Diese Wandstärke ergibt zum einen einen genügend großen elektrischen Widerstand je Längeneinheit und gewährleistet zum anderen noch eine Bearbeitung des Rohrs in Richtung Formgebung.

Das erfindungsgemäß eingesetzte Heizrohrweist eine Wärmekapazität von weniger als 2,5 J/K auf. Hierzu ist im Beispielsfall das eingesetzte Rohr etwa 60 cm lang und weist einen Innendurchmesser von etwa 3 mm auf.

Zur besseren Vermischung der zu erwärmenden Flüssigkeit ist das eingesetzte Heizrohr U-förmig gebogen und weist im Beispielsfall einen Biegeradius von etwa 4 cm auf, wobei die Schenkel des U-förmigen Rohrs etwa gleich lang und parallel zueinander angeordnet sind.

Als Material für das Rohr wird ein physiologisch unbedenklicher Werkstoff eingesetzt, beispielsweise Edelstahl, Titan und dergl., das auch gegenüber korrosiven Sterilisationsmedien inert ist. Vorteilhafterweise wird ein kalt nachgezogenes Edelstahlhalbzeug als Rohrmaterial eingesetzt, wobei vorzugsweise die Wandstärke auf ± 10 % eingehalten wird.

Ein derartiges Rohr gewährleistet beim erfindungsgemäßen Betrieb ein gleichförmiges Temperaturprofil, wobei der höchste Temperaturwert am stromab liegenden Ende des Rohrs an der Innenwandoberfläche des Rohrs erzielt wird. Bekanntermaßen ergeben sich in Abhängigkeit vom Medium und von der Strömungsgeschwindigkeit sowie den geometrischen Größen des Rohrs unterschiedliche Strömungsformen (laminare oder turbulente Strömung), die den Wärmetransport und damit auch die Temperatur der Rohrwandoberfläche direkt beeinflussen.

Zur Verbesserung der Konvektion einer laminaren Strömung ist dabei - wie vorstehend erläutert - das Rohr U-förmig gestaltet. Hierdurch wird im Bereich der Rohrkrümmung eine Sekundärströmung erzeugt, welche die konvektive Wärmeleitung verbessert.

Zur Verbesserung der Durchmischung der Flüssigkeit ist das Heizrohr an den geraden Schenkeln, d.h. den geraden Teilbereichen, derart bearbeitet, daß es auf der Innenoberfläche eine erhabene Form aufweist. Vorteilhafterweise ist die erhabene Form spiralförmig ausgebildet, die dadurch erzeugt wird, daß von außen her in die Rohrwand eine schraubenförmige Sicke eingedrückt wird. Diese Sicke erzeugt dann im Inneren des Rohrs eine spiralförmige Einbuchtung /Erhebung, wodurch die parallelen Bahnen der Flüssigkeitsteilchen bei laminarer Strömung aufgebrochen werden.

Vorteilhafterweise wird diese spiralförmige erhabene Form an den Endbereichen bzw. in den Bereichen der Schenkel fortgeführt, an denen Meßfühler zur Bestimmung der Temperatur angeordnet sind. Hierdurch soll durch den verbesserten Wärmetransport nach dem Verlassen der aktiven Heizzone eine zuverlässige Temperaturmessung gewährleistet werden.

Nahezu den gleichen Effekt erzeugen weitere Verformungen wie zum Beispiel tropfenförmige Einbuchtungen, welche entweder an den gegenüberliegenden Seiten eingebracht werden oder jeweils um 90° versetzt, auf einer umlaufenden Bahn in die Rohrwand eingepreßt werden.

Ein Ausführungsbeispiel wird anhand der Zeichnung erläutet.

Es zeigen:
- Fig. 1: eine prinzipielle Ansicht eines Heizrohr,
- Fig. 2: die Anordnung des Heizrohrs in einer Bedieneinheit in Verbindung mit einer Sensoreinheit in prinzipieller Ansicht und
- Fig. 3: ein Schaltbild einer Steuereinheit zur Steuerung der Heizleistung und Überwachung des Systems.

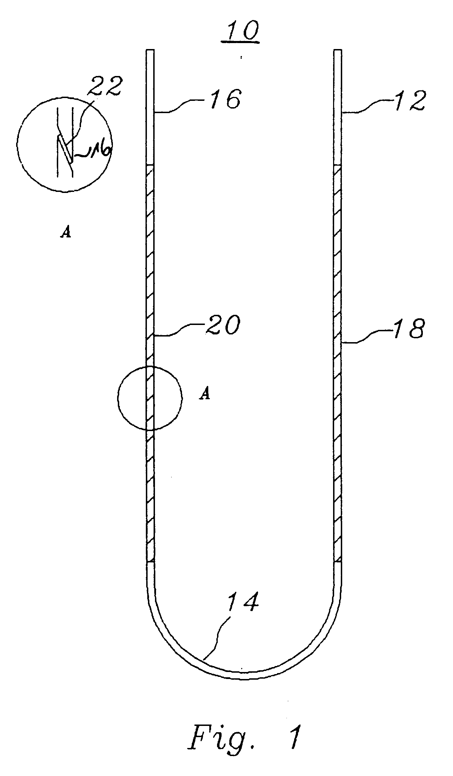

In Figur 1 ist mit 10 ein aus Metall bestehendes Heizrohr bezeichnet, das U-förmig ausgebildet ist. Dieses Heizrohr 10 besteht aus einem Anströmschenkel 12, einem sich an den Anströmschenkel 12 anschließenden U-förmigen Bereich 14 und dem sich daran anschließenden Abströmschenkel 16.

Beide Schenkel 12 und 16 sind in etwa gleich lang und weisen einen Sickenbereich 18 bzw. 20 auf, der spiralförmig ausgebildet ist und auf der Rohrinnenseite eine erhabene spiralförmige Struktur 22, wie im Ausschnitt von Fig. 1 gezeigt, hinterläßt. Die Ansicht A ist eine Längsschnittansicht durch den Abströmschenkel 16, wobei eine Halbwindung der erhabenen Struktur 22 gezeigt ist.

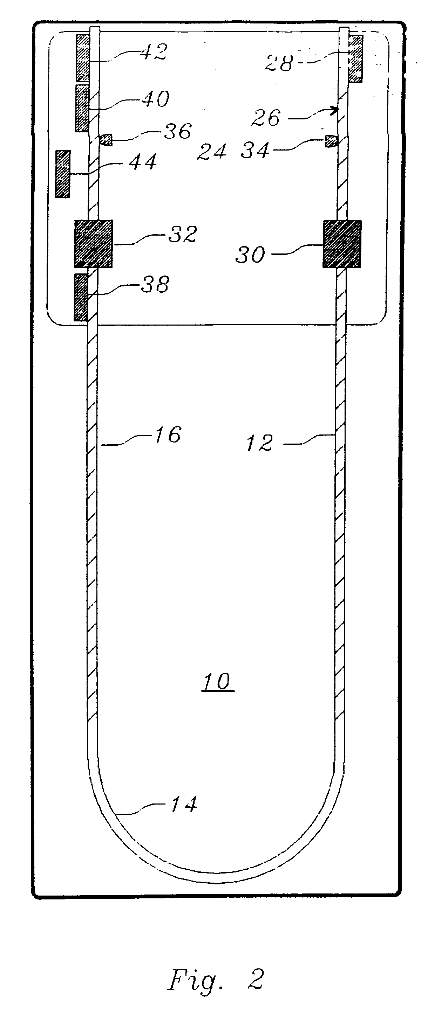

In Figur 2 ist die Montage des Heizrohrs 10 in eine Bedieneinheit 24 gezeigt, wobei die Anschlußleitungen zur Zuführung und Abführung der zu erwärmenden Flüssigkeit aus Übersichtlichkeitsgründen nicht gezeigt sind. Der Anströmschenkel 12 weist in seinem Einströmbereich 26 einen ersten Temperatursensor 28 auf, mit dem die Temperatur der einströmenden Flüssigkeit bestimmt wird.

Hieran schließt sich eine elektrische Klemme 30 und dieser gegenüberliegend im Abströmschenkel 16 eine weitere elektrische Klemme 32 an, an denen Wechselstrom zur Energiezuführung angeschlossen ist.

Desweiteren sind an den beiden Schenkeln Kontaktstifte 34 bzw. 36 vorgesehen, mit denen die Güte der elektrischen Konnektierung, die richtige Lage und der bestimmungsgemäße elektrische Widerstand des Rohres 10 bestimmt werden kann.

Unmittelbar stromauf der zweiten elektrischen Klemme 32 am Abströmschenkel 16 ist ein zweiter Temperatursensor 38 vorgesehen, mit dem die maximale Rohrwandungstemperatur bestimmt und überwacht werden kann. Stromab der zweiten elektrischen Klemme 32 schließt sich dann ein dritter Temperatursensor 40 zur Bestimmung und Regelung der mittleren erreichten Flüssigkeitstemperatur an, der weiter stromab gefolgt ist von einem vierten Temperatursensor 42, mit dem der maximale Grenzwert der mittleren Flüssigkeitstemperatur überwacht werden kann. Alle diese Sensoren sind zur Temperaturmessung an die Außenwand des Heizrohres 10 unter Federbelastung angekoppelt.

Schließlich ist noch in der Bedieneinheit 24 ein fünfter Temperatursensor 44 vorgesehen, mit dem die Umgebungstemperatur in unmittelbarer Nähe des dritten und vierten Temperatursensors 40, 42 ermittelt werden kann, die dann zur Korrektur von Meßfehlern verwendet wird.

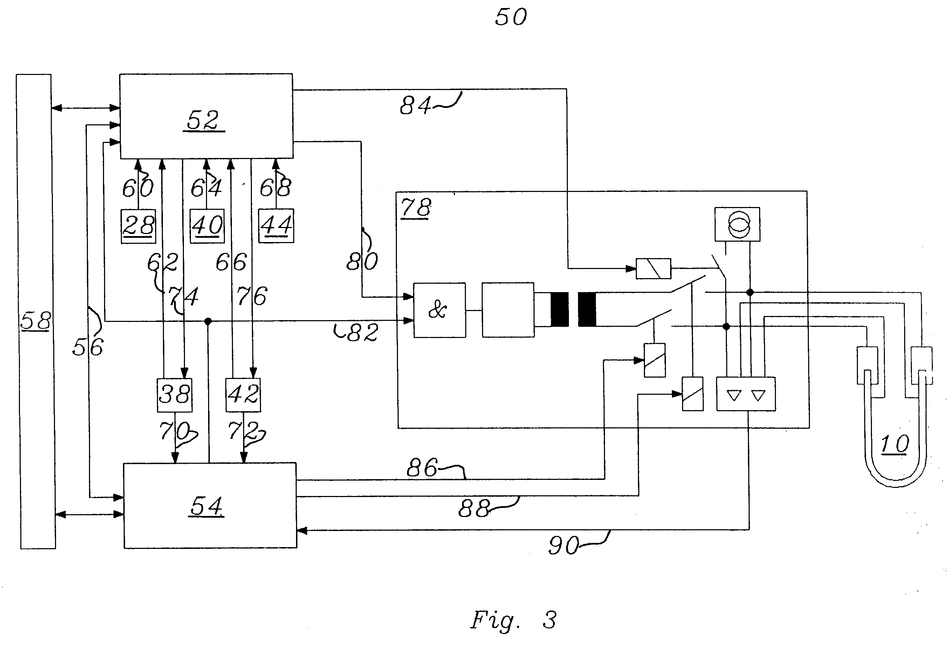

In Figur 3 ist ein Blockschaltbild einer Steuerungseinrichtung 50 dargestellt, die im wesentlichen aus einem Betriebssystem 52 und einem Schutzsystem 54 besteht, die üblicherweise jeweils durch einen Mikroprozessor gebildet sind, die über eine gemeinsame Leitung 56 sich gegenseitig überwachen. Das Betriebssystem 52 und das Schutzsystem 54 sind weiterhin mit einer Eingabe - und Recheneinheit 58 verbunden.

Der erste, dritte und fünfte Temperatursensor 28, 40, 44 ist über je eine Leitung 60, 64 und 68 mit dem Betriebssystem 52 verbunden. Desweiteren sind der zweite und vierte Temperatursensor 38 und 42 über Leitungen 70 und 72 mit dem Schutzsystem 54 verbunden und geben ebenfalls an dieses ihre Werte ab. Desweiteren können der zweite und der vierte Temperatursensor 38 bzw. 42 vom Betriebssystem 52 über die Leitungen 74 und 76 über den festen Alarmwert bestimmt werden, damit das Schutzsystem 54 im Falle eines Initialtests den zweiten und vierten Temperatursensor 38 und 42 prüfen kann.

Die Steuereinrichtung 50 weist weiterhin eine Leistungssteuerungseinheit 78 auf, in der die in das Heizrohr 10 einzuspeisende elektrische Energie zur Verfügung gestellt wird.

Diese Leistungssteuerungseinheit 78 ist mit dem Betriebssystem 52 über eine Steuerleitung 80 verbunden, über die das Aktivierungssignal abgegeben wird.

Weiterhin ist die Leistungssteuerungseinheit 78 über eine weitere Steuerleitung 82 mit dem Steuerteil 52 und dem Schutzsystem 54 verbunden, über das der Desaktivierungsbefehl erteilt wird.

Weiterhin ist das Betriebssystem 52 über die Leitung 84 mit einer Klemmenprüfvorrichtung verbunden. Diese prüft initial mittels der Kontaktstifte die Güte der elektrischen Konnektierung. Das Ergebnis der Prüfung wird über die Datenleitung 90 dem Schutzsystem 54 mitgeteilt.

Schließlich sind zwei Abschaltleitungen 86 und 88 zwischen dem Schutzsystem 54 und der Leistungssteuerungseinheit 78 vorgesehen, mit der Relais zur Abschaltung des Stromflusses im Fehlerfall aktiviert werden.

Das Heizrohr 10 wird mittels der elektrischen Klemmen 30, 32 an die Leistungssteuerungseinheit 78 angeschlossen. Die Versorgungsspannung wird dabei mit geringen Übergangswiderständen an das Heizrohr 10 angekoppelt. Der gesamte sekundäre Stromkreis ist in der Leistungssteuerungseinheit 78 als floatendes Anwendungsteil ausgeführt.

Die Steuerung der Heizleistung in der Leistungssteuerungseinheit 78 erfolgt nach dem Prinzip der Vollwellensteurung. Dabei wird über das Verhältnis der Ein- und Ausschaltzeiten die erforderliche Leistung eingestellt. Um Gleichstromanteile durch den in der Leistungssteuerungseinheit 78 vorgesehenen Transformator zu vermeiden, beträgt die netzsynchrone Einschaltzeit immer ganzzahlige Vielfache von 20 ms.

Das für die Leistungssteuerung vorgesehene Betriebssystem als Steuerteil 52 verarbeitet neben der aktuellen Flußrate, die über die Eingabeeinheit 58 eingegeben oder aber von einem nicht gezeigten Durchflußsensor übermittelt werden kann, die Temperaturwerte der Temperatursensoren nach einem vorbestimmten Programm, wobei die Grenzwertmeßfühler 38 und 42 den sicheren Betrieb der Heizvorrichtung gewährleisten.

Um die in Abhängigkeit von der Umgebungstemperatur auftretenden Meßfehler an den Temperatursensoren auszugleichen, wird zusätzlich noch von dem Steuerteil 52 das Signal des fünften Temperatursensors 44 verabeitet.

Das Schutzsystem 54 tritt u.a. dann in Kraft, wenn die Grenzwerte der Temperatursensoren 38 und/oder 40 überschritten werden, der Verriegelungshebel der Bedieneinheit betätigt wird oder nicht erlaubte Steuerbefehle über die Schnittstelle 58 eingegeben werden.

Tritt ein solcher erster Fehler auf, so überführt das Schutzsystem 54 die gesamte Anordnung in den sicheren Zustand, wobei sämtliche Energiequellen von dem Heizrohr 10 abgetrennt werden.

Mit der erfindungsgemäßen Vorrichtung können zu applizierende Flüssigkeiten sicher und schnell auf die gewünschte Temperatur in unterschiedlichen Flußraten erwärmt werden.

## Patentansprüche

1. Vorrichtung zum Erwärmen von medizinischen Flüssigkeiten mit einem Metallrohr (10), das einen Anströmschenkel (12), einen U-förmigen Bereich (14) und einen Abströmschenkel (16) aufweist und von der Flüssigkeit durchströmbar ist, mit einer elektrischen Energiequelle, die mit dem Metallrohr (10) verbunden ist, und mit einer Steuereinheit (50), welche die elektrische Energiequelle steuert,
**dadurch gekennzeichnet**,
daß das Metallrohr (10) auf der Rohrinnenseite in Teilbereichen eine erhabene Form aufweist, das Metallrohr (10) eine derart dünne Wand aufweist, daß die Wärmekapazität höchstens 2,5 J/K beträgt, das Metallrohr (10) elektrisch direkt beheizt wird, daß am Metallrohr (10) im Einströmbereich (26) des Anströmschenkels (12) ein erster Temperatursensor (28) zur Bestimmung der Temperatur der einströmenden Flüssigkeit und im Bereich des Abströmschenkels (16) wenigstens ein weiterer Temperatursensor (40) zur Bestimmung der mittleren Flüssigkeitstemperatur vorgesehen sind, und daß im Umgebungsbereich des Zuletzt genannten Temperatursensors (40) ein weiterer Temperatursensor (44) zur Bestimmung der Umgebungstemperatur vorgesehen ist, wobei die Steuereinheit (50) die in das Metallrohr (10) einzuspeisende Energie entsprechend dem von den Temperatursensoren (28,40,44) ermittelten Istwert und einem vorgegebenen Sollwert nachführt.

2. Vorrichtung nach Anspruch 1, **dadurch gekennzeichnet**, daß am Anströmkanal (12) anschließend an den ersten Temperatursensor (28) eine erste elektrische Klemme (30), und dieser gegenüberliegend am Abströmschenkel (16) eine zweite elektrische Klemme (32) angeordnet ist, wobei unmittelbar stromauf der zweiten elektrischen Klemme (32) ein weiterer Temperatursensor (38) zur Bestimmung der maximalen Rohrwandtemperatur und stromab der zweiten elektrischen Klemme (32) der Temperatursensor (40) zur Bestimmung der mittleren Flüssigkeitstemperatur und weiter stromab ein vierter Temperatursensor (42) zur Bestimmung der maximalen Grenztemperatur angeordnet sind.

3. Vorrichtung nach Anspruch 1, **dadurch gekennzeichnet**, daß die Steuereinheit (50) ein Betriebsteil (52) und ein Schutzteil (54) aufweist, wobei das Betriebsteil (52) entsprechend dem von dem ersten und den zwei weiteren Temperatursensoren (28,40,44) vorgegebenen Istwert und dem mittels einer Eingabeeinheit (58) vorgegebenen Sollwert eine Leistungssteuerungseinheit (78) regelt und das Schutzteil (54) bei Überschreitung von Grenzzuständen die Vorrichtung in den sicheren Zustand überführt.

## Claims

1. Device for heating medical fluids having a metal tube (10), which has an in-flow limb (12), a U-shaped region (14) and an out-flow limb (16) and the fluid flows through it, having an electrical energy source, which is connected to the metal tube (10), and having a control unit (50) which controls the electrical energy source, characterised in that the metal tube (10) has a raised shape on the inner side of the tube in part regions, the metal tube (10) has a thin wall such that the heat capacity is 2.5 J/K at the most, the metal tube (10) is electrically heated directly, in that a first temperature sensor (28) is provided on the metal tube (10) in the in-flow region (26) of the in-flow limb (12) to determine the temperature of inflowing fluid and at least one further temperature sensor (40) is provided in the region of the out-flow limb (16) to determine the average fluid temperature, and in that a further temperature sensor (44) to determine the ambient temperature is provided in the surrounding region of the last-mentioned temperature sensor (40), wherein the control unit (50) monitors the energy supplied to the metal tube (10) according to the actual value determined by the temperature sensors (28, 40, 44) and a preset theoretical value.

2. Device according to claim 1, characterised in that a first electrical terminal (30) is arranged on the in-flow channel (12) connected to the first temperature sensor (28) and a second electrical terminal (32) is arranged opposite the first on the out-flow limb (16), wherein a further temperature sensor (38) for determining the maximum tube wall temperature is arranged directly upstream of the second electrical terminal (32) and temperature sensor (40) for determining the average fluid temperature is arranged downstream of the second electrical terminal (32) and a fourth temperature sensor (42) for determining the maximum limiting temperature is arranged further downstream.

3. Device according to claim 1, characterised in that the control unit (50) has an operating part (52) and a protection part (54), wherein the operating part (52) controls a power control unit (78) according to the actual value preset by the first and the two further temperature sensors (28, 40, 44) and the theoretical value preset by means of an input unit (58), and the protection part (54) transfers the device into the safer state when limiting states are exceeded.

## Revendications

1. Dispositif servant à réchauffer des fluides médicaux au moyen d'un tube métallique (10), comportant une branche d'arrivée du flux (12), une zone en U (14) et une branche de départ du flux (16), et susceptible d'être parcouru par le fluide, comprenant une source d'énergie électrique qui est reliée au tube métallique (10) et une unité de commande (50) qui commande la source d'énergie électrique, caractérisé en ce que le tube métallique (10), sur sa face intérieure, présente, de façon localisée, une forme convexe, en ce que le tube métallique (10) présente une paroi d'une finesse telle que la capacité thermique représente au maximum 2,5 J/K, en ce que le tube métallique (10) est chauffé directement de façon électrique, en ce que sur le tube métallique (10), dans la zone d'entrée du flux (26) de la branche d'arrivée du flux (12) sont prévus un premier capteur de température (28) destiné à déterminer la température du liquide arrivant et, dans la zone de la branche de départ du flux (16), au moins un autre capteur de température (40) destiné à déterminer la température moyenne du liquide, et en ce que, dans la zone environnante de ce dernier capteur de température (40), un autre capteur de température (44) est prévu pour déterminer la température ambiante, l'unité de commande (50) restituant l'énergie destinée à être transmise au tube métallique (10) en fonction de la valeur réelle détectée par les capteurs de température (28, 40, 44) et d'une valeur de consigne prédéterminée.

2. Dispositif selon la revendication 1, caractérisé en ce que sont disposées, sur le canal d'arrivée (12), après le premier capteur de température (28), une première borne électrique (30) et, en regard de celle-ci, sur la branche de départ du flux (16), une deuxième borne électrique (32), un autre capteur de température (38) étant disposé, immédiatement en amont de la deuxième borne électrique (32), afin de déterminer la température maximale de la paroi du tube et, en aval de la deuxième borne électrique (32), le capteur de température (40), destiné à déterminer la température moyenne du fluide et, encore en aval, un quatrième capteur de température (42) destiné à déterminer la température limite maximale.

3. Dispositif selon la revendication 1, caractérisé en ce que l'unité de commande (50) présente un organe d'exploitation (52) et un organe de protection (54), l'organe d'exploitation (52) régulant une unité de commande de puissance (78), en fonction de la valeur réelle prédéterminée par le premier et les deux autres capteurs de température (28, 40, 44) et en fonction de la valeur de consigne prédéterminée au moyen d'une unité d'entrée de données (58), et l'organe de protection (54) faisant passer le dispositif à l'état de sécurité lorsque sont dépassées des conditions limite.
